**Europäisches Patentamt**

**European Patent Office**

**Office européen des brevets**

(19)

(11) Veröffentlichungsnummer: **0 205 865**

**B1**

(12) # EUROPÄISCHE PATENTSCHRIFT

(45) Veröffentlichungstag der Patentschrift:
21.06.89

(21) Anmeldenummer: **86106367.5**

(22) Anmeldetag: **09.05.86**

(51) Int. Cl.⁴: **A 61 K 31/54 //**
**(A61K31/54, 31:505, 31:275)**

(54) **Pharmazeutische Präparate mit antihypertensiver und kardioprotektiver Wirkung.**

(30) Priorität: **17.05.85 DE 3517820**

(43) Veröffentlichungstag der Anmeldung:
**30.12.86 Patentblatt 86/52**

(45) Bekanntmachung des Hinweises auf die Patenterteilung:
**21.06.89 Patentblatt 89/25**

(84) Benannte Vertragsstaaten:
**AT BE CH LI LU NL SE**

(56) Entgegenhaltungen:
**US-A-4 157 394**

**UNLISTED DRUGS, Band 31, Nr. 9, September 1979,
Seite 135, Chatham, N.J., US;
UNLISTED DRUGS, Band 21, Nr. 9, September 1969,
Seite 142, Chatham, N.J., US;
UNLISTED DRUGS, Band 35, Nr. 5, Mai 1983, Seite
76, Chatham, N.J., US;
ROTE LISTE, 1982, Editio Cantor, Aulendorf/Würrt.,
DE;**

(73) Patentinhaber: **Röhm Pharma GmbH, Dr.- Otto-
Röhm- Strasse 2-4, D-6108 Weiterstadt 1 (DE)**

(72) Erfinder: **Lossnitzer, Klaus, Dr., Zur Gräth 2,
D-6635 Schwalbach- Hülzweiler (DE)**
Erfinder: **Ober, Karl- Friedrich, Dr., Mainstrasse 7,
D-6100 Darmstadt (DE)**
Erfinder: **Giebenhain, Gerhard, Dr.,
Bordenbergweg 15, D-6109 Mühltal (DE)**
Erfinder: **Völger, Karl Dieter, Dr., Beebelstrasse
10, D-6101 Bickenbach (DE)**
Erfinder: **Zeiss, Gerhard, Dr., Sudetenstrasse 12c,
D-6080 Gross- Gerau (DE)**

EP 0 205 865 B1

**Beschreibung**

Gebiet der Erfindung

Die Erfindung betrifft ein pharmazeutisches Präparat mit antihypertensiver und kardioprotektiver Wirkung unter Verwendunung eines Calcium-Antagonisten in Kombination mit dem Kaliumsparer Triamteren und dem Diuretikum Hydrochlorothiazid in fester Kombination.

Stand der Technik

Aus dem US-A-4 157 394 sind pharmazeutische Präparate mit kardioprotektiver Wirksamkeit bekannt, die calciumantagonistische Wirkstoffe, insbesondere das Verapamil in Kombination mit Triamteren enthalten. Diese Präparate zeigen an sich interessante Eigenschaften, beispielsweise eine verstärkte Wirkung der aktiven Bestandteile.

Aufgabe

Es besteht weiterhin besonders im Hinblick auf eine erwünschte Standardtherapie das Bedürfnis nach einem Präparat mit deutlich diuretischen und gleichzeitig kardioprotektiven Eigenschaften. Soweit die Wirkstoffe Verapamil und Triamteren in derartige Überlegungen einbezogen wurden, schienen doch eine Reihe von Argumenten dagegen zu sprechen:

Verapamil ist ein Blutdrucksenker. Die üblicherweise angewendeten Dosen sind relativ hoch (240 mg Tagesdosis). Die Tagesdosen werden daher üblicherweise aufgeteilt oder in Retardform eingenommen (vgl. "Tote Liste"). Triamteren wird (überwiegend in Form eines Kombinationspräparats mit dem Diuretikum Hydrochlorothiazid) ebenfalls als Blutdrucksenker eingesetzt. Die üblichen, zur Blutdrucksenkung empfohlenen Dosen liegen bei 1 - 2 Tabletten pro Tag (mit - im allgemeinen - 50 mg Triamteren plus 25 mg Hydrochlorothiazid pro Tablette.)

Lösung

Zur Lösung der bestehenden Aufgabe eignet sich das erfindungsgemäße Kombinationspräparat in besonderem Maße.

Das erfindungsgemäße antihypertensive und kardioprotektive Kombinationspräparat umfaßt als Wirkstoffe die Verbindung 2-Isopropyl-2,8-bis(3,4 dimethoxyphenyl)-6-methyl-6-azaoctannitril ("Verapamil") in Form ihrer pharmakologisch unbedenklichen Säureadditionssalze, speziell des Hydrochlorids 2,4.7-Triamino-6-phenyl-pteridin ("Triamteren") sowie 6-Chlor-3,4-dihydro-7-sulfamoyl-2H-1,2,4-benzothiadiazin-1,1-diozid ("Hydrochlorothiazid"). Unte pharmakologisch unbedenklichen Säureadditionssalzen seien beispielsweise die Salze der d-Weinsäure, Maleinsäure, Fumarsäure, Bernsteinsäure, Zitronensäure, Zimtsäure, Salicylsäure, Adipinsäure, Essigsäure, Propionsäure, p-Aminobenzoesäure, Methansulfonsäure, Schwefelsäure, Phosphorsäure, insbesondere die Hyrochlorid und Lactate verstanden.

Vorzugsweise ist die erfindungsgemäße Kombination der drei Wirkstoffe Bestandteil einer Dosierungseinheit, vorzugsweise einer Tablette, speziell im (ungefähren bis exakten) Gewichtsverhältnis Verapamil in Form eines pharmakologisch undbedenklichen Säureadditionssalzes zu Triamteren zu Hydrochlorthiazid wie 16 : 5 : 2,5, besonders bevorzugt im Verhältnis 160 mg : 50 mg : 25 mg.

Somit wird eine Dosierung zur Verfügung gestellt, die 160 mg Verapamil-Hydrochlorid, 50 mg Triamteren und 25 mg Hydrochlorthiazid in Form einer Tablette als Tagesdosis enthält. Daneben ist auch die Verabreichung in Kapsel- bzw. Drageeform möglich. Die neuen pharmazeutischen Präpate können enteral verabreicht werden, bevorzugt ist jedoch die orale Verabreichung. Die Präparate können die üblichen Träger- und Hilfsstoffe enthalten.

Die bevorzugte Ausführungsform der Erfindung stellen somit feste, zur oralen Verabreichung geeignete Zubereitungen dar, insbesondere Tabletten. Als Trägermaterialien können pharmazeutisch indifferente Feststoffe, wie beispielsweise Mannit, Milchzucker, organische oder anorganische Calciumsalze etc. wervendet werden.

Als Bindemittel kommen u.a. Polyvinlylpyrrolidon, Gelatine oder Cellulosederivate infrage. Als weitere Zusätze können Tablettensprengmittel, wie beispielsweise Stärke oder Alginsäure, Gleitmittel, wie z. B. Stearinsäure oder deren Salze und anorganische Fließmittel, wie z. B. Talk oder kolloidale Kieselsäure, sowie Geschmackskorrigenten, verwendet werden.

Die Wirkstoffe können mit den Hilfsstoffen in üblicher Weise gemischt und naß oder trocken granuliert werden. Je nach Art der verwendeten Zusatzstoffe kann gegebenenfalls auch durch einfaches Mischen ein direkt tablettierbares Pulver erhalten werden. Das Granulat oder Pulver kann direkt in Kapseln abgefüllt oder vorzugsweise in üblicher Weise zu Tablettenkernen verpreßt werden.

Die Erfindung umfaßt somit ein Verfahren zur Behandlung des Bluthochdrucks bei dem als Tagesdosis eine Tablette verabreicht wird, die 160 mg Verapamil-Hydrochlorid, 50 mg Triamteren und 25 mg Hydrochlorothiazid, enthält.

Die erfindungsgemäße Kombination hat eine über die Wirkung der Einzelkomponenten hinausgehende therapeutische Wirkung. Hervorzuheben ist, daß diese Wirkung mit den relativ niedrigen Einmaldosen der Einzelkomponenten, nämlich 160 mg Verapamil-Hydrochlorid 50 mg Triamteren und 25 mg Hydrochlorothiazid hervorgerufen werden kann.

Die Herstellung von Tabletten, welche die erfindungsgemäße Wirkstoffcombination in einer Ein-Tagesdosierung enthalten, kann wie folgt vorgenommen werden:
Die Herstellung der Tabletten kann zweckmäßig in mehreren Abschnitten erfolgen:

A. Herstellung von Verapamil-Granulat (60,4 % Verapamil-Hydrochlorid)

Zur Herstellung von 100 kg Verapamil-Granulat benötigt man:

| | |
|---|---|
| Verapamil-Hydrochlorid | 60,38 kg |
| ®Aerosil pyrogene Kieselsäure | 0,51 kg |
| Mikrocristalline-Zellulose (®Avicel PH 102) | 19,10 kg |
| Calcium-Carboxymethylcellulose | 5,00 kg |
| (®Emcompress) Tablettierungshilfsstoff aus Dialciumphosphat | 10,00 kg |
| Magnesiumstearat | 1,51 kg |
| Talkum | 3,50 kg |

Verapamil-Hydrochlorid, ®Aerosil, ®Avicel PH 102, Calciumcarboximethylzellulose sowie ®Emcompress und Talkum werden in einem Mischer (Lödigemischer FKM 130) 5 Minuten/Messerkreuz gemischt, gesiebt über 0,9 mm Maschenweite, danach Zugabe von Magnesiumstearat. Weitere 10 Minuten mischen mit Messerkreuz. Anschließend Kompaktierung auf einem Kompaktor.

Bei Walzeneinstellung 9, Materialdosierschnecke 8 - 9, wird auf dem Hutt-Kompaktor unter einem Walzendruck von 70 - 80 KN die Masse verdichtet. Hierbei stellt sich eine Temperatur von ca. 35°C des Kompaktats ein. Anschließend granuliert man mit einem Granuliersieb mit der Maschenweite 0,9 mm. Das Granulat weist dann in der Fließprobe relativ gute Fließfähigkeit auf: 4 - 8 Sek. Durchflußprobe 7 - 8 mm. Schüttdichte 6,3 - 6,9 g/ml. Verapamilgehalt 60,38 % gefunden, 97,2 - 103,5 % des Solls.

B. Herstellung einer Triamteren-Hydrochlorothiazid-Tablettenmasse

Zur Herstellung der Tablette nach C dient die folgende Rezeptur (50 kg-Ansatz):

| | |
|---|---|
| Triamteren | 12,500 kg |
| Hydrochlorothiazid | 6,250 kg |
| Calciumcarbonat | 1,860 kg |
| pyrogene Kieselsäure (®Aerosil) | 0,410 kg |
| Maisstärke | 2,660 kg |
| Lactose K | 2,160 kg |
| Milchzucker-Granulat | 13,200 kg |
| Polyvinylpyrrolidon (mittl. MG 25 000) | 0,390 kg |
| Magnesiumstearat | 0,550 kg |
| Mikrokristalline Zellulose | 4,820 kg |
| Carboxymethylstärke | 1,000 kg |
| Talkum | 4,200 kg |
| | 50,000 kg |

C. Herstellung einer Triamteren-Hydrochlorothiazid-Verapamil-Hydrochlorid-Tablette (Verhältnis 50 mg 25 mg : 160 mg)

Das Verapamil-Granulat nach A. und die Tablettenmasse nach B. im angegebenen Verhältnis werden gesiebt und 15 Minuten im Rhönradmischer gemischt. Anschließend wird auf einer Rundlauftablettenpresse mit einem Werkzeugdurchmesser von 10 mm facettiert und ohne Bruchkerbe bei 35 Umdrehungen pro Minute und einem Preßdruck von 8 - 10 KN abgepreßt; man erhält Tabletten mit einem Gewicht von 465 mg.

Die Effektivität der erfindungsgemäßen Wirkstoffkombination geht aus der folgenden Doppelblindstudie hervor. Es wurden insgesamt 9 Behandlungsgruppen zu je 24 Patienten gebildet. Bei den Patienten handelte es sich um Hypertoniker, die nach einer zweiwöchentlichen Placebophase einen systolischen Blutdruck von 160 - 180 mm Hg und einen diastolischen Butdruck von 95 - 120 mm Hg aufwiesen. Nach der Placebophase erhielten die Patienten über drei Wochen hinweg einmal morgens in randomisierter Zuordnung entweder

1. Placebo
2. 80 mg Verapamil-Hydrochlorid (V)
3. 160 mg Verapamil-Hydrochlorid (V)
4. 12,5 mg Hydrochlorothiazid (HCT) und 25 mg Triamteren (TA)
5. 25 mg Hydrochlorothiazid (HCT) und 50 mg Triamteren (TA)

6.     12,5 mg HCT, 25 mg TA, 80 mg V
7.     12,5 mg HCT, 25 mg TA, 160 mg V
8.     25 mg HCT, 50 mg TA, 80 mg V
9.     25 mg HCT, 50 mg TA, 160 mg V

Der systolische und diastolische Blutdruck wurde morgens vor der Arzneimittelgabe, nach 3 Minuten im Liegen und nach 1 Minute im Stehen gemessen.

Die Verabreichung der Wirkstoffe geschah in Tablettenform (in Anlehnung an ("Herstellung der Tabletten" s. oben).

Aus der folgenden Tabelle ist die ausgeprägte antihypertensive Wirkung der Triamteren-Hydrochlorothiazid-Verapamil-Hydrochlorid Kombination bei Einmaldosierung festzustellen, wobei die Kombination 25 mg HCT, 50 mg TA und 160 mg V den günstigsten Effekt hatte.

Die Kombination aus 25 mg HCT und 50 mg TA wird als "Dytide H" bezeichnet.

**Tabelle 1**
Mittlere Blutdruckveränderungen inden verschiedenen Behandlungsgruppen

Messung im Liegen (syst./diast.)

| Behandlungs-gruppe | Ende Placebo-phase (mmHg) | Ende Behand-lung (mmHg) | % Veränderung (%) |
|---|---|---|---|
| Placebo | 174,6/101,4 | 169,8/99,2 | -2,7/-2,2 |
| Verapamil 80 mg | 169,0/100,2 | 164,2/98,8 | -2,9/-1,3 |
| Verapamil 160 mg | 175,3/104,7 | 159,7/97,5 | -8,9/-6,9 |
| 1/2 Dytide H | 175,6/105,4 | 176,8/102,8 | +0,7/-22,5 |
| 1/1 Dytide H | 171,4/103,2 | 165,6/100,0 | -3,4/-3,2 |
| 1/2 Dytide H + Verapamil 80 mg | 172,8/100,5 | 155,5/91,8 | -10,0/-8,6 |
| 1/2 Dytide H + Verapamil 160 mg | 175,2/104,5 | 155,3/88,5 | -11,3/-15,3 |
| 1/1 Dytide H + Verapamil 80 mg | 176,0/104,7 | 158,0/94,5 | -10,2/-9,7 |
| 1/1 Dytide H + Verapamil 160 mg | 174,5/105,7 | 151,7/89,7 | -13,1/-15,1 |

Messung im Stehen (syst./diast.)

| Behandlungs-gruppe | Ende phase (mmHg) | Behand-lung (mmHg) | % Veränderung (%) |
|---|---|---|---|
| Placebo | 172,8/100,0 | 169,8/100,6 | -1,7/+0,6 |
| Verapamil 80 mg | 171,0/100,0 | 166,5/99,7 | -2,6/-0,3 |
| Verapamil 160 mg | 177,3/105,0 | 162,7/97,0 | -8,3/-7,6 |
| 1/2 Dytide H | 177,6/106,0 | 175,8/102,4 | -1,0/-3,4 |
| 1/1 Dytide H | 170,6/103,6 | 165,8/99,2 | -2,8/-4,7 |
| 1/2 Dytide H + Verapamil 80 mg | 172,3/100,7 | 156,0/93,3 | -9,5/-7,3 |
| 1/2 Dytide H + | | | |

| | | | |
|---|---|---|---|
| Verapamil 160mg | 173,8/103,7 | 157,5/90,3 | -9,4/-12,9 |
| 1/1 Dytide H + Verapamil 80 mg | 174,5/105,3 | 156,5/94,2 | -10,3/-10,6 |
| 1/1 Dytide H + Verapamil 160 mg | 172,0/103,0 | 152,0/90,7 | -11,6/12,0 |

## Patentansprüche

1. Pharmazeutisches Präparat mit antihypertensiver und kardioprotektiver Wirkung, dadurch gekennzeichnet, daß es 2-Isopropyl-2,8-bis(3,4-dimethoxyphenyl)-6-methyl-6-azaoctannitril (Verapamil) in Form eines pharmakologisch unbedenklichen Säureadditionssalzes, 2,4,7-Triamino-6-phenyl-pteridin (Triamteren) und 6-Chlor-3,4-dihydro-7-sulfamoyl-2H-1,2,4-benzothiadiazin-1,1-dioxid (Hydrochlorothiazid) als Wirkstoffe neben den üblichen Trägern bzw. Hilfsstoffen in Form einer einzigen Verabreichungseinheit enthält.

2. Pharmazeutisches Präparat gemäß Anspruch 1, dadurch gekennzeichnet, daß es 2-Isopropyl-2,8-bis(3,4-dimethoxyphenyl)-6-methyl-6-azaoctannitril (Verapamil) in Form eines pharmakologisch unbedenklichen Säureadditionssalzes, Triamteren und Hydrochlorotriazid als Wirkstoffe im Gewichtsverhältnis 16 : 5 : 2,5 in Form einer einzigen Verabreichungseinheit enthält.

3. Pharmazeutisches Präparat gemäß den Ansprüchen 1 und 2, dadurch gekennzeichnet, daß es 2-Isopropyl-2,8-bis(3,4-dimethoxyphenyl)-6-methyl-6-azaoctannitril (Verapamil) in Form des Hydrochlorids enhält.

4. Pharmazeutisches Präparat gemäß den Ansprüchen 1 bis 3, in einer oral zu verabreichenden Form.

5. Pharmazeutisches Präparat gemäß den Ansprüche 1 bis 5, dadurch gekennzeichnet, daß es 160 mg Verapamil-Hydrochlorid 50 mg Triamteren und 25 mg Hydrochlorothiazid enthält.

7. Pharmazeutisches Präparat gemäß Anspruch 6, dadurch gekennzeichnet, daß es die Wirkstoffe in Form einer einzigen Dosierungseinheit enthält.

## Claims

1. A pharmaceutical preparation with an antihypertensive and cardioprotective activity, characterized in that it contains 2-isopropyl-2,8-bis(3,4-dimethoxyphenyl)-6-methyl-6-azaoctannitrile (verapamil) in the form of a pharmacologically acceptable acid addition salt, 2,4,7-triamino-6-phenyl-pteridine (triamterene) and 6-chloro-3,4-dihydro-7-sulphamoyl-2H-1,2,4-benzothiadiazine-1,1-dioxide (hydrochlorothiazide) as active substances together with conventional carriers and/or excipients in the form of a single dosage unit.

2. A pharmaceutical preparation as claimed in claim 1, characterized in that 2-isopropyl-2,8-bis(3,4-dimethoxyphenyl)-6-methyl-6-azaoctannitril (verapamil) in the form of a pharmacologically acceptable acid addition salt, triamterene and hydrochlorothiazide as active substances are present in a ratio by weight of 16 : 5: 2.5.

3. A pharmaceutical preparation as claimed in claim 1 and claim 2, charaterized in that 2-isopropyl-2,8-bis(3,4-dimethoxyphenyl)-6-methyl-6-azaoctannitril (verapamil) is contained in the form of its hydrochloride.

4. A pharmaceutical preparation, as claimed in claim 1 to 7, in a form for oral administration.

5. A pharmaceutical preparation as claimed in claims 1 to 4, in tablet form.

6. A pharmaceutical preparation as claimed in claims 1 to 5 characterized in that it contains 160 mg of verapamil, 50 mg of triamterene and 25 mg of hydrochlorothiazide.

7. A pharmaceutical preparation as claimed in claim 6 characterized in that it contains the active substances in the form of a single dosage unit.

## Revendications

1. Préparation pharmaceutique à action anti-hypertensive et cardioprotectrice, caractérisée en ce qu'elle contient, sous la forme d'une unique unité posologique, de 1: α-isopropyl-α-[(N-méthyl-N-homovératryl)-κ-aminopropyl]-3,4-diméthoxyphényl-acétonitrile sous forme d'un sel d'addition d'acide acceptable pharmacologiquement, de la 2,4,7-triamino-6-phényl-ptéridine (triamtérène) et du 6-chloro-3,4-dihydro-7-sulfamoyl-2H-1,2,4,-benzothiadiazine-1,1-dioxyde (hydrochlorothinzide) en tant que substances actives, en dehors des excipients ou adjuvants usuels.

2. Préparation pharmaceutique selon la revendication 1, caractérisée en ce qu'elle contient, sous la forme d'une unique unité posologique, de l'α-isopropyl-α-[(N-méthyl-N-homovératryl)-γ-aminopropyl]-3,4-

diméthoxyphényl-acétonitrile sous forme d'un sel d'addition d'acide acceptable pharmacologiquement, du triamtérène et de l'hydrochlorothiazide en tant que substances actives dans le rapport en poids 16 : 5 : 2,5.

3. Préparation pharmaceutique selon la revendication 1 ou 2, caractérisée en ce qu'elle contient l'81-isopropyl-α-[(N-méthyl-N-homovératryl)-γ-aminopropyl]-3,4-diméthoxyphényl-acétonitrile sous la forme de son chlorhydrate (vérapamil).

4. Préparation pharmaceutique selon l'une quelconque des revendications 1 à 3, sous une forme administrable par voie orale.

5. Préparation pharmaceutique selon l'une quelconque des revendications 1 à 4, sous forme de comprimés.

6. Préparation pharmaceutique selon l'une quelconque des revendications 1 à 5, caractérisée en ce qu'elle contient 160 mg de vérapamil, 50 mg de triamtérène et 25 mg d'hydrochlorothiazide.

7. Préparation pharmaceutique selon la revendication 6, caractérisée en ce qu'elle contient les substances actives sous la forme d'une unique unité posologique.